# EUROPEAN PATENT APPLICATION

(11) **EP 3 766 405 A1**
(43) Date of publication of application: **20.01.2021**
(21) Application number: 19193554.3
(22) Date of filing: 26.08.2019
(51) Int. Cl.: A61B 3/10, A61B 5/00

(54) **METHOD AND APPARATUS FOR INVESTIGATING A SAMPLE**

(30) Priority: 15.07.2019 EP 19186383
(71) Applicant: ETH Zurich, 8092 Zurich (CH)
(72) Inventor: GÖKSEL, Orcun, 8044 Zürich (CH); KLING, Sabine, 8053 Zürich (CH)

(57) **Abstract**

The method for investigating a sample, e.g., a biological sample, comprises:
- carrying out (110) a first optical coherence measurement in a first probe volume of the sample while the sample is exposed to an ambient pressure amounting to a pressure p1 to obtain first measurement data (210); subsequently
- changing (120) an ambient pressure to which the sample is exposed from the pressure p1 to a pressure p2 which is different from the pressure p1; subsequently
- carrying out (130) a second optical coherence measurement in a second probe volume to obtain second measurement data (220);
- carrying out (140) an evaluation in dependence of both, the first (210) and the second (220) measurement data.

The method makes possible to detect, under physiological conditions, biomechanical properties of materials inside the sample and/or functional properties of the sample.

## Description

The invention relates to the field of non-destructive examination. It can be of particular interest for application to biological material, ex vivo and in-vitro, respectively, but also in-vivo. The invention relates to methods and apparatuses according to the opening clauses of the claims. It can find application, e.g., in investigations in mechanical properties of tissues or other biological materials.

From US 2014/275935 A1, an inflatable mask is known, which comprises two ocular cavities which can seal against a user's face by forming an air-tight seal around the periphery of the user's eye socket. The sealed air-tight ocular cavity can be pressurized to take ocular measurements. The mask can conform to the contours of a user's face by inflating or deflating the mask. The distance between the user and a medical device (e.g., an optical coherence tomography instrument) can be adjusted by inflating or deflating the mask.

In US 2017/061621 A1, an automated tissue stiffness measurement device is presented. According to the description, the device can identify cancerous lesions with high sensitivity and specificity. More particularly, systems and methods are presented there for measuring tissue stiffness using applied force, illumination and imaging techniques. The systems and methods can use structured illumination to characterize a tissue surface. Said US 2017/061621 A1 aims at detecting skin deseases and in particular skin cancer. In various embodiments, a form of elastography can be implemented to simulate manual palpation and estimate the stiffness of the lesion.

In ophthalmology, a known way of estimating intraocular pressure is to use a tonometer, e.g., for detecting glaucoma at an early stage. Besides contact-based methods, there is also non-contact tonometry, which is mostly preferred for obvious reasons. It uses a rapid air pulse to locally flatten and/or indent the cornea, which is detected via an electro-optical system.

Furthermore, optical coherence elastography (OCE) is a known imaging technique used in biomedical imaging to form high-resolution pictures of biological tissue, which makes it possible to map biomechanical properties of tissue. OCE has begun to find application in ophthalmology, on the one hand in a contacting way using a gonioscopy lens and on the other hand in a non-contact way, where either pulsed laser light is used to generate surface acoustic waves on the cornea, or the cornea is locally exposed to a short focused micro air puff.

One possible object of the invention is to create a new way of investigating a sample, such as a biological sample, in a non-contact fashion.

One possible object of the invention is to create a way of investigating a sample, such as a biological sample, in which the sample does not need to be exposed to an unnatural stress distribution, in particular in which the sample is exposed to a stimulus which simulates a stress distribution of physiological stimuli.

One possible object of the invention is to create a way of investigating a sample, such as a biological sample, in which the sample does not need to be exposed to an unnaturally high stress to to unnaturally high forces, respectively, in particular in which the sample is exposed to a stimulus which simulates a stress and/or a force having an amplitude corresponding to an amplitude of a stress and/or a force of physiological stimuli.

One possible object of the invention is to create a new way of investigating a sample, such as an eye in-vivo.

One possible object of the invention is to create a new way of investigating a biological sample, in particular a cell culture or a tissue culture or an organ culture in-vitro.

One possible object of the invention is to create a new way of accomplishing elastography measurements.

One possible object of the invention is to create a new way of accomplishing measurements of one or more functional properties of a sample, in particular of a biological sample.

One possible object of the invention is to create a new way of characterizing a sample, in particular a biological sample, which can be helpful in detecting irregularities and/or abnormalities in the sample, such as in biological tissue.

One possible object of the invention is to create a new way of investigating a sample under sterile conditions.

One possible object of the invention is to create a new way of investigating a sample, in which measurements can be synchronized with a stimulus provided by an entity comprising the sample, such as synchronized to a heart beat of a creature comprising the sample or synchronized to a breathing cycle of a creature comprising the sample.

One possible object of the invention is to create a new way of investigating a sample which allows to determine biomechanical properties of the sample, in particular by distunguishing influences of pre-stressing on strain from influences of biomechanical properties of the sample on strain.

One possible object of the invention is to create corresponding methods and apparatuses.

Further objects and various advantages emerge from the description and embodiments below.

At least one of these objects can be achieved in some implementations of apparatuses and/or methods described in this disclosure.

Optical coherence elastography (OCE) is a powerful method for characterizing a sample, in particular a biological sample, with respect to its mechanical properties, such as with respect to its strain. When a sufficient pentration depth for the light used in the OCE measurement is provided, sample characterization deeply into the sample is possible. The stimulus to which the sample, e.g., a human eye, is exposed in order to induce in the sample changes to be detected can be disagreeable or disturbing, in particular when accomplished in contact-mode, such as using a gonioscope. And furthermore, for example when using pulsed laser light and focused air puff stimulation, the stimulus may act on the sample very locally only, thus resulting in unphysiologial stress and thus in results which require a rather complex interpretation, in particular regarding clinical and/or functional relevance of the results.

The inventors contemplated that it can be advantageous and lead to new results and insights to use optical coherence measurements in combination with a stimulus which is, in particular regarding its amplitude (or strength) and/or its stress distribution, close to physiological stimuli, and/or in combination with a stimulus which largely corresponds to a physiological stimulus. By physiological stimuli we mean, in particular, stimuli to which the sample is exposed under natural conditions. XXX

The stimulus to which the sample is exposed in the method can simulate a physiological stimulus with respect to its amplitude or strength. And/or the stimulus to which the sample is exposed in the method can simulate a physiological stimulus with respect to its stress distribution, e.g., provided the sample is naturally exposed to varying isotropic pressure, the stimulus used in the method can be isotropic, too.

The inventors further contemplated that an excellent choice of a stimulus can be the ambient pressure to which the sample is exposed, and moreover that it can be sufficient and lead to useful results if changes in the ambient pressure are small, corresponding to physiological ambient pressure changes and/or to physiological diurnal variations in tissue tension.

Thus, it is suggested to combine optical coherence measurements with ambient pressure changes as a stimulus, in particular wherein the ambient pressure changes are physiologically small.

Accordingly, the method can be a method for investigating a sample, and it can comprise:
(a) carrying out a first optical coherence measurement in a first probe volume of the sample while the sample is exposed to an ambient pressure amounting to a pressure p1 to obtain first measurement data; subsequently
(b) changing an ambient pressure to which the sample is exposed from the pressure p1 to a pressure p2 which is different from the pressure p1; subsequently
(c) carrying out a second optical coherence measurement in a second probe volume to obtain second measurement data;
(d) carrying out an evaluation in dependence of both, the first and the second measurement data.

The method can also be regarded as a method for determining a property of a sample, in particular a mechanical property of the sample, more particularly a biomechanical property of a sample. E.g., the method can also be regarded as an elastographical method or as a method for elastographically examining a sample. And/or it can be regarded as a method for determining or at least assessing a functional property of a sample, wherein said functional property can be, e.g., an optical property, such as a refractive power, as may be the case when investigating an eye, or it can be, e.g., a flow velocity of a fluid flowing in the sample.

The method can make possible to obtain information about a strain and/or of a Poisson's ratio of a sample (or of a portion thereof) while treating the sample relatively gently, in a non-contact fashion, and, with further assumptions about the sample, also a stiffness of the sample (or of a portion thereof) can be determined, at least as an approximation. This can be particularly useful when the method shall be carried out in-vivo. However, also in-vitro investigations can profit from the method, as relatively natural and/or small deformations can be applied to the sample as a stimulus for the measurements.

Moreover, the method can make possible to determine strain in a rather straight-forward manner (and without having to make specific assumptions about the sample). The strain can be determined by two main items: a pre-stress to which the sample is exposed and a stiffness of the sample. The method can furthermore, at least in some instances, make possible to distinguish between these two items, as the change in ambient pressure can simulate a main factor effecting the pre-stress. Thus, the method can make possible to obtain results for both, a stiffness and a pre-stress. And this can be the case without having to make specific assumptions about the sample for obtaining these results.

The method can make possible to investigate material present inside the sample, i.e. materials below a surface of the sample. E.g., interfaces between different materials inside the sample or other sources of light scattering can be investigated and more particularly, positions of such interfaces can be determined, and changes therein which are induced by ambient pressure changes can be detected and can allow to determine strain and/or a Poission's ratio, and further properties of the respective materials, such that properties related to or indicative of elasticity and/or stiffness, can be deduced.

While on the one hand, the proposed method can be particularly helpful in investigations in human eyes and in animal eyes, it can on the other hand also be applied (in-vitro) to cell cultures or to tissue cultures or to organ cultures. The method is, however, not limited to such applications, as any other, e.g., non-biological, sample may be examined, provided that the penetration depth of the light used in the optical coherence measurements is sufficiently large.

The invention comprises apparatuses with features of corresponding methods according to the invention, and, vice versa, also methods with features of corresponding apparatuses according to the invention.

The advantages of the apparatuses basically correspond to the advantages of corresponding methods, and, vice versa, the advantages of the methods basically correspond to the advantages of corresponding apparatuses.

In particular, the apparatus can be an apparatus for investigating a sample, and the apparatus can comprise
- an optical coherence measurement device for carrying out optical coherence measurements in a probe volume of the sample to obtain measurement data;
- an ambient pressure modulator for applying an adjustable ambient pressure to the sample;
- an evaluation unit coupled to the optical coherence measurement device for receiving the measurement data.

The ambient pressure modulator can comprise
- a chamber member establishing a measurement cavity for containing a fluid; and
- a pressure adjuster operationally connected to the chamber member, for adjustment of a pressure of the fluid in the measurement cavity.

Therein, the evaluation unit is configured for carrying out an evaluation in dependence of first measurement data obtained in a first optical coherence measurement in a first probe volume and of second measurement data obtained in a second optical coherence measurement in a second probe volume.

Therein, between the first optical coherence measurement and the second optical coherence measurement, an ambient pressure to which the sample is exposed is changed by the ambient pressure modulator.

The sample can be a sample of material, in particular of biological material.

Therein, biological material can more particularly be a material produced by a biological organism.

The sample can be a biological sample; more particularly in the sense that a biological sample is (or used to be) a part of a biological organism.

In some embodiments, the sample comprises an ensemble of tissues, such as a biological organ. The described method can be particularly well suited for such samples, in particular for in-situ and more particularly for in-vivo measurements of such samples. E.g., the sample can be a human eye, e.g., investigated in-vivo.

In some embodiments, the sample comprises a cell culture. The described method can be particularly well suited for such samples, in particular for in-vitro measurements of such samples.

In some embodiments, the sample comprises a tissue culture. The described method can be particularly well suited for such samples, in particular for in-vitro measurements of such samples.

In some embodiments, the sample comprises an organ culture. The described method can be particularly well suited for such samples, in particular for in-vitro measurements of such samples.

In some embodiments, the sample comprises, in particular the sample comprises in both, the first and the second probe volume, at least two sources of scattering, which are reached by the light used in the optical coherence measurements, and at which said light is scattered sufficiently strongly for making possible to determine their mutual distances from the optical coherence measurements.

For example, in some embodiments, the sample comprises, in particular the sample comprises in both, the first and the second probe volume, at least two layers of material having at least one interface each, which are reached by the light used in the optical coherence measurements, and at which said light is scattered sufficiently strongly for making possible to determine their mutual distances from the optical coherence measurements.

For example, the sample can comprise, in the first and second sample volumes, two or more different tissues forming layers which are stacked upon each other.

Typically, the first and second probe volumes are identical probe volumes, wherein identical of course means identical within the framework of the method, i.e. under consideration of measurement accuracies and stability of the experimental setup.

Usually, the first and second probe volumes are (at least) overlapping. This way, a good comparability of the first measurement data with the second measurement data can be ensured.

E.g., the first and second probe volumes can be strongly overlapping in the sense that they share at least 50% of their respective volumes or, more particularly in the sense that they share at least 75% of their respective volumes

In some embodiments, the first probe volume and/or the second probe volume, in particular both, are rod-shaped volumes. This can be the case when the light beam, more particularly the sample beam, used in the optical coherence measurements is - relative to the sample - in a constant position during the optical coherence measurements. E.g., the optical coherence measurements in this case can be one depth scan each. Such measurements shall be referred to as one-dimensional optical coherence measurements.

In some embodiments, the first probe volume and/or the second probe volume, in particular both, are panel-shaped volumes. This can be the case when the sample beam used in the optical coherence measurements is moved (scanned) along a line, in particular along a straight line, so that the measurement comprises a number of depth scans lying side-by-side along said line. Such measurements shall be referred to as two-dimensional optical coherence measurements.

In some embodiments, the first probe volume and/or the second probe volume, in particular both, are cuboid-shaped volumes. This can be the case when the sample beam used in the optical coherence measurements is moved (scanned) across an area, for example along an array, e.g., along points of a set of mutually parallel straight lines, so that the measurement comprises a number of depth scans distributed over an area, e.g., forming an array. Such measurements shall be referred to as three-dimensional optical coherence measurements.

The optical coherence measurements can be accomplished by an optical coherence measurement device, e.g., a Fourier-domain optical coherence measurement device.

The optical coherence measurement device can be a commercially available optical coherence tomography (OCT) apparatus.

Since OCT is a well known technique, it will not be necessary to go into details here, so that only some specific points will be mentioned in this regard.

The optical coherence measurement device can comprise a light source for emitting light, a beam splitter and a mirror, for separating the light into a reference beam and a sample beam, and for reuniting the (returning) sample beam and the reference beam so as to let them interfer with one another, and a light detector for detecting the resulting interference signal, such as a photodiode or a photodetector array, e.g., for spectrally separated detection.

The light source can be provided for emitting light of different wavelengths, e.g., for emitting light having a continuous (or possibly quasi-continuous) wavelengths spectrum. For example, the wavelengths of the spectrum are emitted simultaneously, such as when the light source is a superluminiscent diode (SLED). Or, in another example, the wavelengths of the spectrum are generated at different times, e.g., by sweeping the wavelengths of the momentarily emitted light, such as when the light source comprises a tunable or, more precisely, wavelength scannable laser. Or, in still another example, the wavelengths of the spectrum are generated because the light is emitted in ultrashort light pulses (laser pulses).

The emitted light can have a stable phase. In order to accomplish this, the light source can comprise a phase stabilizer, in particular in case of a swept source, such as described above.

The wavelengths of the emitted light can be comprised in the infrared portion of the electromagnetic spectrum, e.g., they can have wavelengths between 780 nm and 5000 nm. In particular, the wavelengths of the emitted light can be comprised in the near-infrared portion of the optical spectrum, e.g., have wavelengths between 780 nm and 1400 nm. Infrared light is not too strongly absorbed by many biological materials; in other words, it has a sufficient penetration depth for many (biological) samples. However, depending on the sample, also light contained in a different part of the electromagnetic spectrum can be used.

Optical coherence measurements are based on low-coherence interferometry.

Light (of a sample beam) scattered inside the sample, e.g., at interfaces inside the sample or at other sources of light scattering, is made to interfere with a reference beam, such that depth information, more particularly information about interfaces or about sources of light scattering inside the sample, is obtained.

As has been sketched above already, the optical coherence measurements can be one-, two-, or three-dimensional measurements. The one-dimensional optical coherence measurements do not need to comprise a lateral scanning of the sample beam. Lateral refers to directions perpendicular to the light beam direction (in the sample), which is referred to as the z-direction (depth direction). Such measurements can be useful in the herein described method, while not constituting OCT measurements.

The two-dimensional optical coherence measurements involve a one-dimensional lateral scanning of the sample beam, i.e. a scanning along a line, e.g., along a straight line, such as scanning of the sample beam along the x-direction.

The three-dimensional optical coherence measurements involve a two-dimensional lateral scanning of the sample beam, i.e. a scanning along points of an array, such as scanning of the sample beam along the x- and the y-direction.

(The x-, y-, and z-directions are meant to describe a Cartesian coordinate system.)

Other scanning / imaging techniques can also be used, such as full-field OCT or linear OCT or flying sopt.

In case lateral scanning of the sample beam is to be accomplished, the optical coherence measurement device comprises a light scanning mechanism, such as one or more controllably movable mirrors.

The measurement data obtainable in the optical coherence measurements can have a dimensionality which scales with the dimensionality of the optical coherence measurements. For each dimension, the measurement data can comprise, e.g., one or both of intensity information, phase information (each in dependence of the depth).

By exposing the sample to a different pressure (e.g., changing from pressure p1 during the first optical coherence measurement to pressure p2), a slight deformation of the sample can be provoked which can lead to slight deformations or displacements of constituents of the sample, wherein an amount of these deformations or displacements depends on a stiffness of the respective constituents involved. E.g., less stiff parts of the sample can exhibit a larger deformation or displacement than stiffer parts of the sample. Detection of auxetic material properties is possible, too.

Considering that scattering intensity and/or phases of light of the sample beam at sources of light scattering inside the sample (such as at interfaces in the sample) can be detected by the optical coherence measurements, said deformations or displacements can be quantified from the optical coherence measurements.

A particularly high sensitivity regarding detection of displacements and a particularly high spatial resolution in detecting strain can be obtained if phase information in the measurement data is evaluated. When spectrally resolved light detection is accomplished using an array detector, e.g., by a one-dimensional (line-) detector, subpixel displacements of sources of light scattering can be readily achieved from information of phase information. This can facilitate detecting small changes taking place in the sample in reaction to the change in ambient pressure.

The change in ambient pressure from pressure p1 to pressure p2 is a stimulus to induce changes in the sample, such as deformations or displacements.

By changing the ambient pressure to which the sample is exposed, the sample is exposed to a uniformly distributed stimulus, the stimulus is an isotropic (hydrostatic) pressure; one and the same pressure acts on any part of the exposed surface of the sample. This is in contrast to local stimuli such as focused air puffs, which mainly induce shear stresses. In many cases, such a uniformly distributed stimulus is closer to stimuli occurring to a sample under natural conditions, such that findings detected in reaction to ambient pressure changes can have a particularly high significance, e.g., compared to results from local stimuli.

The ambient pressure changes can be accomplished using an ambient pressure modulator for applying an adjustable ambient pressure to the sample. The ambient pressure modulator can comprise a chamber member establishing a measurement cavity for containing a fluid and a pressure adjuster operationally connected to the chamber member, for adjustment of a pressure of the fluid in the measurement cavity.

Prior to step (a), the method can comprise a step (e) of adjusting an ambient pressure to which the sample is exposed to pressure p1. This step (e) can be accomplished, e.g., by the ambient pressure modulator. This step may be unnecessary in case pressure p1 is identical with an atmospheric pressure which is present at the location and at the time of carrying out the first optical coherence measurement.

The fluid conveying the ambient pressure can be a gas, but in instances, it can be a liquid.

For example, the fluid can be air. Or the fluid can be an inert gas. Or the fluid can be oxygen (O₂).

Inside the chamber member and more particularly inside the measurement cavity, the ambient pressure is present to which the sample is exposed.

In some embodiments, the chamber member and more particularly the measurement cavity has an opening to be closed, e.g., by the sample or by a periphery connected to the sample, more particularly by a peripheral region adjacent the sample, or in a combined fashion partially by the sample and partially by said periphery or peripheral region. Connecting to a peripheral region can effect that boundary effects are negligible. E.g., in case of in-vivo measurements of a human eye as a sample, the opening can be closed by skin of the respective person's face.

In some embodiments, the measurement cavity has a proximal opening circumferentially delimited by a sealing member for sealing a closed volume jointly defined by the measurement cavity and a peripheral region adjacent the sample, by forming a circumferential sealing connection between the sealing member and the peripheral region. This way, a closed volume can be established which is filled with the fluid having the desired pressure (such as p1 or p2). The volume can be closed in a fluid-tight manner. Fluid-tight means impermeable by fluids, i.e. by gases and liquids, more specifically impermeable by the fluid in the measurement cavity; it can also be gas-tight and, more specifically, air-tight.

Such chamber members and measurement cavities, respectively, can be particularly useful, e.g., for in-situ and for in-vivo investigations, e.g., of biological samples, more particularly of biological tissues.

The sealing member can be provided for inhibiting flow of the fluid into or out of the measurement cavity. It can be made of, e.g., a resilient fluid-tight material, such as a silicone.

In some embodiments, the chamber member and more particularly the measurement cavity completely encloses a volume. E.g., two mutually sealing, separable parts can be provided in order make the inside of the measurement cavity accessible, such as for inserting the sample. Such embodiments can be particularly useful for in-vitro measurements, e.g., of biological material, and in particular of cell cultures or tissue cultures or organ cultures.

In some embodiments, the chamber member is rigid and dimensionally stable, respectively. If the chamber member comprises a part having a sealing function, such as the sealing member, that part may optionally be not rigid and dimensionally stable, respectively. If the chamber member is not dimensionally stable, it may become possible that the ambient pressure undergoes unwanted changes.

In some embodiments, the chamber member has a port for letting fluid flow into the measurement cavity and/or letting flow fluid out of the measurement cavity, as controlled by the pressure adjuster.

In some embodiments, the chamber member comprises a light passage member for letting the sample beam and light of the wavelength range used in the optical coherence measurements, respectively, enter the measurement cavity through the chamber member and exit the measurement cavity through the chamber member. E.g., the light passage member can comprise a window of a material which is transparent for the light of the wavelength range used in the optical coherence measurements. Or it can be a fluid-tight feedthrough, e.g., for an optical fiber for guiding the sample beam. However, if, e.g., the light is generated inside the chamber member, there may be no need for a light passage member.

In some embodiments, the light passage member is made of a rigid material. Deformations of the light passage member could easily lead to false or inconsistent or irreproducible or otherwise corrupted measurements and results, respectively.

The light passage member can comprise an anti-reflective coating for minimizing reflections; in particular in case the light passage member comprises a window.

The light passage member can comprise an optical lens for deflecting, e.g., focusing the sample beam; in particular in case the light passage member comprises a window. It can comprise two or more such optical lenses, e.g., for enabling, in a relatively simple way, different guidances for the sample beam.

In some embodiments, the method comprises, in addition the steps of
(a') carrying out a third optical coherence measurement in a third probe volume of the sample while the sample is exposed to an ambient pressure amounting to the pressure p1 to obtain third measurement data; subsequently
(b') changing an ambient pressure to which the sample is exposed from the pressure p1 to the pressure p2; subsequently
(c') carrying out a fourth optical coherence measurement in a fourth probe volume to obtain fourth measurement data;
wherein the evaluation is carried out in further dependence of the third and the fourth measurement data; and
wherein the first and second optical coherence measurements are carried out with a reference beam incident on the sample at an angle of incidence which is different from an angle of incidence with which the third and fourth optical coherence measurements are carried out.

Doing so can make possible to distinguish different directional strain components in the sample. In other words, while it is usually possible, with the sample beam impining on the sample under one single angle of incidence only and thus following one single beam direction only, to investigate or determine strain components directed along that one beam direction only, the method with steps (a'), (b'), (c') as depicted above makes possible to investigate or determine two different strain components, namely one which is directed along a first beam direction (according to said first angle of incidence) and another one which is directed along a second beam direction (according to said second angle of incidence). From said two strain components, one can deduce, e.g., two strain components which are components along mutually perpendicular directions.

The first and the second and the third and the fourth can be mutually overlapping probe volumes.

In some embodiments, the pressure adjuster comprises a pressure transducer for determining the ambient pressure near the sample. For example, the pressure transducer can be in fluid communication with the fluid in the measurement cavity, e.g., the pressure transducer can be located in the measurement cavity, such as close to or adjacent to the proximal opening.

In some embodiments, the pressure adjuster comprises a pressure changer for changing the ambient pressure to which the sample is exposed.

The pressure changer can comprise, e.g., a valve and/or a pump, and it can comprise a fluid reservoir bearing a portion of the fluid separate from the fluid in the measurement cavity.

In some embodiments, the pressure changer is connected to a port of the chamber member. The port can enable an exchange of fluid between the measurement cavity and the outside of the chamber member. E.g., the port can be an opening in the chamber member which is closable by means of a valve.

The pressure changer can comprise, e.g., an actuator acting on a deformable section of the chamber member, so as to thereby increase or decrease a volume of the measurement cavity. When keeping the amount of fluid inside the measurement cavity constant, the ambient pressure inside the measurement cavity can be changed this way.

In some embodiments, the pressure adjuster comprises a control circuit. The control circuit can be configured to reach and/or maintain a target pressure (of the fluid) in the measurement chamber and/or to effect a preset march of pressure over time, such as a pressure profile rising linearly in time from a start pressure to an end pressure within a preset time span. More generally, the control circuit can be configured to adjust the pressure according to specifications. The specifications can be, e.g., preset default specifications, e.g., stored in the apparatus, e.g., in the control circuit, or specifications inputted by a user.

In some embodiments, the ambient pressure is adjusted, e.g., by the control circuit, in dependence of an evaluation result, e.g., as obtained in step (d). For example, one can adjust the ambient pressure so as to induce in the sample a strain having a predetermined (preset) amplitude.

The control circuit can be operationally connected to the pressure transducer and to the pressure changer, in particular so as to control the pressure changer in dependence of pressure values determined by the pressure transducer. For example, the control circuit can control the pressure changer in dependence of specifications, to adjust the ambient pressure (i.e. the pressure of the fluid in the measurement cavity) according to the specifications, based on pressure values determined by the pressure transducer. This can be accomplished, e.g., in a closed-loop control.

Reproducibility can be an important point in the investigations. The optical coherence measurements usually shall be reproducible, which includes that the ambient pressures at which the measurements are taken should be reproducible. Therefore, it can be of advantage to provide that a precision in adjusting the ambient pressure amounts to 0.5 mbar or less, in particular to 0.1 mbar or less. This can be helpful for ensuring that a difference between the pressures p1 and p2 is well reproducible.

In some embodiments, pressures p1 and p2 differ from one another by less than 20 mbar, in particular by less than 16 mbar, still more particularly by less than 13 mbar. Corresponding pressure differences correspond to physiological pressure changes and thus to physiological stimuli.

A time span lapsing between the first and the second optical coherence measurement can be, e.g., smaller than 10 s, more particularly smaller than 2 s and even more particularly smaller than 0.5 s.

However, it can be valuable, e.g., in investigating slowly developing changes, to be able to compare measurements, more particularly evaluation results based on respective first and second optical coherence measurements, which are taken with a long time span in between, such as with at least one day in between or with at least four weeks in between. And also, a comparability between different samples, such as between eyes of different human beings, can be valuable.

In particular for such cases, it can be of advantage to provide that an accuracy in adjusting the ambient pressure amounts to 0.5 mbar or less, in particular to 0.1 mbar or less. This can be helpful for ensuring that evaluation results as described are well reproducible and long-term developments can be monitored, and that evaluation results obtained at different samples can be meaningfully compared.

Another aspect regarding reproducibility and quality of evaluation results has been mentioned above already and concerns that the first probe volume and the second probe volume should be identical or at least have a large overlap. To achieve this can be difficult, in particular in case of in-vivo measurements, such as in case of in-vivo measurements at a human eye. Depending on the sample, the sample can be susceptible to involuntary motion and/or to voluntary motion, both possibly detrimentally influencing the evaluation results.

This can be particularly important regarding a pair of first and second optical coherence measurements (taken quickly one after the other), buy may have some importance regarding comparability of evaluation results where the respective pairs of first and second optical coherence measurement are obtained with a long time span in between, as described above.

A sample-to-beam aligner can be helpful in this regard. The sample-to-beam aligner can effect, e.g., that a probe volume in which the first and second optical coherence measurements are overlapping is maximized.

In order to accomplish this, for example one or more of the following can be done by a sample-to-beam aligner, e.g., controlled by a control unit of the apparatus:
- tracking a motion of the sample and carrying out the second optical coherence measurement at a time when a position of the sample deviates from the position of the sample during the first optical coherence measurement by less than a threshold position difference;
- tracking a motion of the sample and steering a sample beam used in the second optical coherence measurement to interact with the sample in a location coinciding, within a threshold location difference, with a location in which the sample beam did interact with the sample during the first optical coherence measurement;
- influencing a motion of the sample to make the sample return towards (and in particular into) the position it had during the first optical coherence measurement.

For example, the first two exemplary possibilities can make use of, e.g., an eye tracker or a similar device which determines a position of an item such as of an eye, e.g., based on image processing. Eye trackers are commercially available devices. The third exemplary possibility, when the method is applied in-vivo to an eye of a person, can be based on, e.g., a visible item such as a light point, wherein the person is asked to firmly look at that visible item, with the goal that the person positions his/her eye reproducibly in one and the same position. Corresponding technologies are widely used in ophthalmology for a long time.

As has been explained, the evaluation of the first and second optical coherence measurements is accomplished in a combined fashion. Therefore, it will usually be the case that the evaluation result changes when the first measurement data are changed, and that the evaluation result also changes when the second measurement data are changed.

An evaluation result can be based on and/or can depend on differences between the first measurement data and the second measurement data.

An evaluation result can make possible to determine changes in the sample, in particular regarding positions of constituents of the sample such as of interfaces in the sample, which depend on and, in particular originate from, changes in ambient pressure, more particularly which depend on and/or originate from the change in ambient pressure from pressure p1 to pressure p2.

In some embodiments, the evaluation comprises obtaining, in particular obtaining from distinctions distinguishing the first measurement data from the second measurement data, one or more properties of the sample, more particularly one or more properties of one or more components of the sample, such as of layers of the sample. Said properties can in particular biomechanical properties, which can relate to, e.g., strains, to Poisson's ratios, to stiffnesses and/or to deformabilities. Or, as depicted further above, they can relate to functional properties.

In other words, in the evaluation, one or more properties of one or more components of the sample can be deduced from the first and second optical coherence measurements, in particular wherein the first and second optical coherence measurements are interrelated with one another in the evaluation.

For example, a displacement of each of one or more portions, e.g., tissue layers, of the sample can be determined in the evaluation. And in the evaluation, from these one or more displacements, e.g., a property indicative of a stiffness or indicative of a deformability or indicative of an elasticity can be determined for one or more of the one or more portions of the sample.

Consequently, the method can also be a method for determining a property of at least one component of the sample.

The evaluation can be accomplished by an evaluation unit of the apparatus.

In a first measurement mode, the second optical coherence measurement is carried out while an ambient pressure to which the sample is exposed amounts to pressure p2. This way, in the evaluation, a state of the sample when exposed to a first pressure (p1) can be compared to or interrelated with a state of the sample when exposed to a second pressure (p2).

In a second measurement mode, the second optical coherence measurement is carried out while an ambient pressure to which the sample is exposed amounts to pressure p1. This way, in the evaluation, a state of the sample when exposed to a first pressure (pi) is compared to or interrelated with a state of the sample when exposed to the same pressure (P1), but provided that the sample has been exposed in the meantime to a second pressure (p2). This way, hysteresis effects can be investigated.

Of course, several other measurement modes can be thought of, e.g., wherein the second optical coherence measurement is carried out while an ambient pressure to which the sample is exposed amounts to pressure p3 differing from both, pressures p1 and p2.

In some embodiments, p2 > p1 applies.

In some embodiments, p1 > p2 applies.

In some embodiments, the apparatus is configured to enable that both, p2 > p1 and p1 > p2 can be accomplished.

In some embodiments, pressure p1 is the atmospheric pressure which is present at the location and at the time of carrying out the first optical coherence measurement.

In other embodiments, pressure p2 is the atmospheric pressure which is present at the location and at the time of carrying out the second optical coherence measurement.

In some embodiments, the steps (a), (b) and (c) are carried out in an automated fashion, in particular one after the other in the order (a), (b), (c). This way, the first and second measurement data can be readily obtained and can be rapidly obtained. No action from a user needs to be required, however, it can be provided that the user initiates the process and optionally preselects one or more of pressure p1, pressure p2, a value for p1 minus p2. If initially, step (e) (adjusting the ambient pressure to p1; cf. above) is carried out, it is optionally possible to provide that steps (e), (a), (b) and (c) are carried out in an automated fashion, in particular one after the other in the order (e), (a), (b), (c).

In some embodiments, the steps (a), (b), (c) and (d) are carried out in an automated fashion, in particular one after the other in the order (a), (b), (c), (d). This way, the evaluation results can be readily obtained and can be rapidly obtained. No action from a user needs to be required, however, it can be provided that the user initiates the process and optionally preselects one or more of pressure p1, pressure p2, a value for p1 minus p2. If initially, step (e) (adjusting the ambient pressure to p1; cf. above) is carried out, it is optionally possible to provide that steps (e), (a), (b), (c) and (d) are carried out in an automated fashion, in particular one after the other in the order (e), (a), (b), (c), (d).

With regard to the apparatus, such automatizations can be implemented, e.g., by providing that the apparatus comprises a control unit which is configured to control the optical coherence measurement device and the ambient pressure modulator in such a way that
(A) a first optical coherence measurement in a first probe volume of the sample is carried out by the optical coherence measurement device to obtain first measurement data, while the sample is exposed to an ambient pressure amounting to a pressure p1; and subsequently
(B) an ambient pressure to which the sample is exposed is changed by the ambient pressure modulator from the pressure p1 to a pressure p2 which is different from the pressure p1; and subsequently
(C) a second optical coherence measurement in a second probe volume of the sample is carried out by the optical coherence measurement device to obtain second measurement data.
   Optionally, the control unit can furthermore be configured to control the evaluation unit in such a way that subsequently,
(D) an evaluation in dependence of both, the first and the second measurement data, is carried out by the evaluation unit.

It can furthermore be optionally provided that the control unit is configured to control the ambient pressure modulator in such a way that prior to the first optical coherence measurement (cf. (A)) the ambient pressure modulator adjusts an ambient pressure to which the sample is exposed to pressure p1.

Automatizations like the ones described above can also contribute to a higher quality of the evaluation results, as the time from the beginning of the first optical coherence measurement (step (a)) and from adjusting the ambient pressure to p1 (step (e)), respectively, to the time when the second optical coherence measurement (step (c)) is finished and the evaluation (step (d)) is finished, respectively, can be very short. Thus, for samples which tend to exhibit motion, such as is often the case for in-vivo investigations, it can be easier to achieve a strong overlap between the first probe volume and the second probe volume, as the sample has less time during which it may exhibit motion.

As will have become clear, methods and apparatuses which are herein described make possible to examine samples, in particular biological samples, so as to detect effects resulting from exposing the sample to different ambient pressures, in particular to pressures simulating physiological pressures and pressure differences, and to determine therefrom information about, e.g., stiffnesses of one or more constituents of the sample, such as of layers of the sample. This can be helpful, e.g., in health-related investigations in various areas, such as in ophthalmology, and also in investigations in cell cultures or in tissue cultures or in organ cultures (e.g., in-vitro).

The methods and the apparatuses can make possible to detect, under physiological loading conditions, biomechanical properties of materials inside the sample.

Further embodiments and advantages emerge from the following description and the enclosed figures and from the dependent claims.

Below, the invention is described in more detail by means of examples and the included drawings. In the drawings, same reference numerals refer to same or analogous elements. The figures show schematically:
- Fig. 1: a schematic illustration of an apparatus;
- Fig. 2: a block diagrammatical illustration of a method.

The described embodiments are meant as examples or for clarifying the invention and shall not limit the invention.

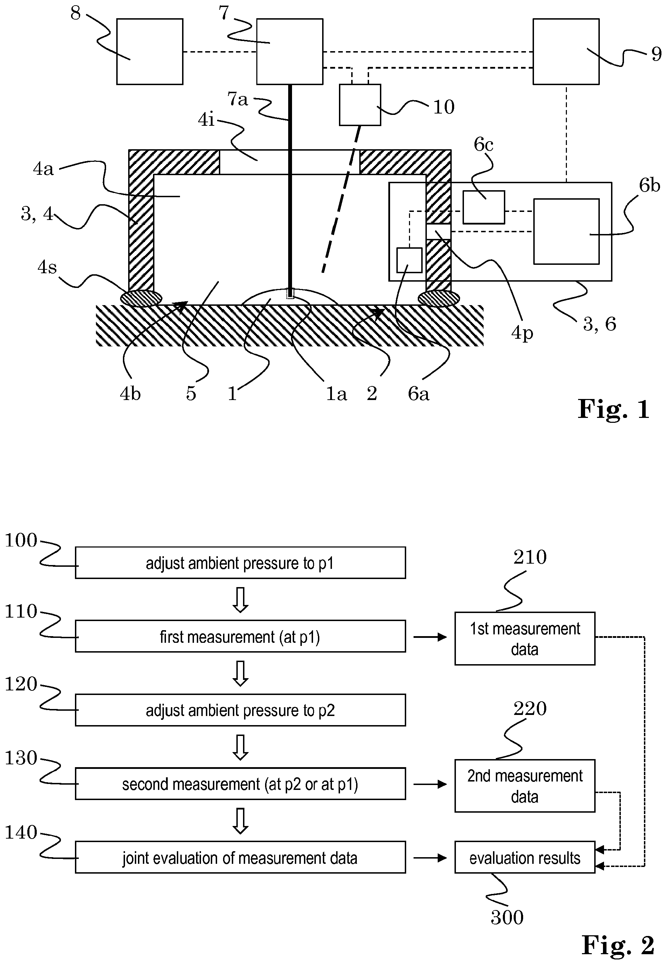

Fig. 1 shows a schematic illustration of an apparatus for investigating a sample 1.

Fig. 2 shows a block diagrammatical illustration of a corresponding method.

The apparatus comprises an ambient pressure modulator 3 comprising a chamber member 4 and a pressure adjuster 6. Optionally, it can comprise a control unit 9, as illustrated in Fig. 1. Dashed lines in Fig. 1 symbolize operational connections.

The chamber member 4 forms a measurement cavity 4a which may comprise a proximal opening 4b and a sealing member 4s delimiting the opening 4b. This way, the measurement cavity 4a can define a volume which is closed in a fluid-tight manner. The measurement cavity 4a can be delimited, in part, by the chamber member 4 and, in part by the sample 1 and a peripheral region 2 adjacent the sample.

For example, the sample 1 can be a person's eye, and the peripheral region 2 can be skin of the face of the person, such as an eye.

In other embodiments (not illustrated in Fig. 1), the chamber member 4 can be designed to fully enclose the measurement cavity 4a itself, in particular in a fluid-tight manner. E.g., the chamber member 4 can comprise two parts which can be in sealing connection but make possible to open the chamber member 4, for inserting the sample 1.

During measurements, the measurement cavity 4acontains, or more particularly is filled with, a fluid 5, such as air or another gas. The sample 1 can thus be exposed to an ambient pressure originating from the fluid 5.

In order to adjust the ambient pressure, the pressure adjuster 6 is provided. In the illustrated example of Fig. 1, the pressure adjuster 6 comprises
- a pressure transducer 6a for determining the ambient pressure in the measurement cavity 4a, which may be (but does not need to be) located inside the measurement cavity 4a;
- a pressure changer 6b for altering the ambient pressure, e.g., a pump; and
- a control circuit 6c operationally connected to both, the pressure transducer 6a and the pressure changer 6b.

The control circuit 6c can control the pressure changer 6b in dependence of pressure values determined by the pressure transducer 6a, and this may be accomplished, e.g., in dependence of settings (specifications) provided by the control unit 9.

As an option, if fluid exchange between the measurement cavity 4a and an outside of the chamber member 4 is required or desired, the chamber member 4 can comprise a port 4p for this purpose. The port 4p is closable, in a fluid-tight manner, e.g., by means of a valve which may be a component of the pressure changer 6b.

The components described above make possible to adjust an ambient pressure to which the sample 1 is exposed to desired values, such as to a pressure p1and afterwards to a pressure p2, as depicted at steps 100 and 120, respectively, in Fig. 2.

The apparatus furthermore comprises an optical coherence measurement device 7 for accomplishing optical coherence measurements, such as depicted at steps 110 and 130 in Fig. 2.

As optical coherence measurements are generally known and have been described further above already, various details in this regard will be omitted in the following.

The optical coherence measurement device 7 can be a commercially available OCT device; however, it is not necessarily required that the sample beam can be scanned (along a line and/or across a surface, such as along points of an array) for a measurement, because optical coherence measurements constituting a single depth scan can be sufficient for carrying out the herein described method. However, it can be provided that the optical coherence measurement device 7 is capable of such a scanning.

If the optical coherence measurement device 7 is located outside the chamber member 4, a light passage member 4i can be provided in the chamber member 4, so as to let a sample beam 7a produced by the optical coherence measurement device 7 enter into the measurement cavity 4a and to let it, or more particularly to let a fraction of the sample beam 7a which is scattered by the sample, leave the measurement chamber 4a along the same path as on the way towards the sample. The light passage member 4i can be, e.g., a transparent region of the chamber member 4, transparent to light of the wavelengths used in the optical coherence measurements.

By the sample beam 7a, a probe volume 1a of the sample 1 is investigated (depicted in Fig. 1 in a strongly schematized manner), as the light enters into the sample 1 with a penetration depth which depends on the properties of the materials from which the sample 1 is composed and on the wavelengths of the light of the sample beam 7a.

Based on interference signals which can be, e.g., intensity signals and/or phase signals characterizing an interference between light of the reference beam (not illustrated) and the returned, scattered sample beam, information about scattering source present inside the respective probe volume can be obtained. The information can be indicative of relative positions of interfaces or other scattering structures, such that corresponding measurement data (cf. items 210 and 220 in Fig. 2) can be jointly evaluated (cf. step 140 in Fig. 2), so as to gain evaluation results (item 300 in Fig. 2) which can be indicative, e.g., of differences between the two measurement data. E.g., if a shift of a first layer present in the sample is larger than a shift of a second layer present in the sample, the respective measurement data can possibly be interpreted as showing that the first and second layers (or respective other scattering sources) have different stiffnesses (or elasticities).

The apparatus comprises an evaluation unit 8 for combined evaluations of first and second measurement data.

In the evaluation unit 8, differences between the first and second measurement data can be considered. The first and second measurement data can be interrelated so as to determine positional changes of layers of different material in the sample.

As shown in Fig. 2, a first optical coherence measurement (step 110) is accomplished while the sample 1 is exposed to an ambient pressure amounting to p1, and afterwards, the sample is exposed to an ambient pressure amounting to p2 (p2 differing from p1).

After the exposure of the sample 1 to the other ambient pressure (p2), a second optical coherence measurement (step 130) is accomplished. E.g., the second optical coherence measurement (step 130) may be accomplished at p2. Thus, the evaluation (step 140) in the evaluation unit (item 7 in Fig. 1) will in this case be based on differences in ambient pressures to which the sample 1 is exposed during the respective measurements.

In another case, a second optical coherence measurement (step 130) may be accomplished after the ambient pressure has returned from pressure p2 back to pressure p1. Thus, the evaluation (step 140) in the evaluation unit (item 7 in Fig. 1) will in this case be based on a hysteresis of changes which happen to the sample in reaction to exposing the sample 1 to a change in ambient pressure.

The control unit 9 can (optionally) be configured to control the respective components of the apparatus (more particularly: the optical coherence measurement device 7 and the pressure adjuster 6) so as to adjust the ambient pressure as desired and to accomplish the optical coherence measurements as described and as illustrated in Fig. 2 Therein, it is generally an option that control unit 9 controls the evaluation unit 8 to carry out step 140 (cf. Fig. 2).

Another option is the provision of a sample-to-beam aligner 10. The sample-to-beam aligner 10 can be configured to control the optical coherence measurement device 7. This may be accomplished directly (as illustrated by the thin dashed line in Fig. 1) or indirectly via the control unit 9. And/or the sample-to-beam aligner 10 can be configured to interact with or to influence the sample 1. The thick dashed line in Fig. 1 symbolizes that sample-to-beam aligner 10 can be configured to image the sample or to interact with the sample. Furthermore, the sample-to-beam aligner 10 can be controlled by the control unit 9 and/or control unit 9 can control sample-to-beam aligner 10.

The inventors discovered that surprisingly, it is possible to detect changes in samples based on small pressure changes, such as where the absolute value of p1 minus p2 is smaller than, e.g., 20 mbar.

For well reproducible results, the precision and optionally also the accuracy with which the ambient pressure modulator adjusts the ambient pressures (p1; p2) should therefore be, e.g., below 1 mbar or rather below 0.5 mbar.

## Claims

1. A method for investigating a sample, in particular a biological sample, the method comprising:
(a) carrying out a first optical coherence measurement in a first probe volume of the sample while the sample is exposed to an ambient pressure amounting to a pressure p1 to obtain first measurement data; subsequently
(b) changing an ambient pressure to which the sample is exposed from the pressure p1 to a pressure p2 which is different from the pressure p1; subsequently
(c) carrying out a second optical coherence measurement in a second probe volume to obtain second measurement data;
(d) carrying out an evaluation in dependence of both, the first and the second measurement data.

2. The method according to claim 1, wherein the changing the ambient pressure is accomplished using an ambient pressure modulator comprising a chamber member and a pressure adjuster operationally connected to the chamber member, wherein the chamber member establishes a measurement cavity containing a fluid establishing the ambient pressure, and wherein a pressure of the fluid in the measurement cavity is adjusted by the pressure adjuster.

3. The method according to claim 1 or claim 2, wherein the pressures p1, p2 are adjusted with a precision of 0.5 mbar or less, in particular of 0.1 mbar or less, more particularly wherein the pressures p1, p2 are adjusted with an accuracy of 0.5 mbar or less, in particular of 0.1 mbar or less.

4. The method according to one of claims 1 to 3, wherein the pressures p1 and p2 differ from one another by less than 20 mbar, in particular by less than 16 mbar, still more particularly by less than 13 mbar.

5. The method according to one of claims 1 to 4, wherein the sample comprises biological material, in particular wherein the sample comprises a cell culture or an ensemble of tissues, more particularly a biological organ.

6. The method according to one of claims 1 to 5, wherein the second optical coherence measurement is carried out while the sample is exposed to an ambient pressure amounting to the pressure p1 or to the pressure p2.

7. The method according to one of claims 1 to 6, further comprising effecting that the first probe volume and the second probe volume are strongly overlapping, in particular by one or more of
- tracking a motion of the sample and carrying out the second optical coherence measurement at a time when a position of the sample deviates from the position of the sample during the first optical coherence measurement by less than a threshold position difference;
- tracking a motion of the sample and steering a sample beam used in the second optical coherence measurement to interact with the sample in a location coinciding, within a threshold location difference, with a location in which the sample beam did interact with the sample during the first optical coherence measurement;
- influencing a motion of the sample to make the sample return towards, in particular into the position it had during the first optical coherence measurement.

8. The method according to one of claims 1 to 7, wherein carrying out the evaluation comprises determining from the first and second measurement data at least one property of one or more constituents of the sample, wherein the at least one property is indicative of a Poisson's ratio, of a stiffness or of a deformability.

9. The method according to one of claims 1 to 8, wherein steps (a), (b) and (c) are carried out in an automated fashion, in particular wherein steps (a), (b), (c) and (d) are carried out in an automated fashion.

10. An apparatus for investigating a sample, in particular a biological sample, the apparatus comprising
- an optical coherence measurement device for carrying out optical coherence measurements in a probe volume of the sample to obtain measurement data;
- an ambient pressure modulator for applying an adjustable ambient pressure to the sample;
- an evaluation unit coupled to the optical coherence measurement device for receiving the measurement data;
the ambient pressure modulator comprising
- a chamber member establishing a measurement cavity for containing a fluid; and
- a pressure adjuster operationally connected to the chamber member, for adjustment of a pressure of the fluid in the measurement cavity;
wherein the evaluation unit is configured for carrying out an evaluation in dependence of first measurement data obtained in a first optical coherence measurement in a first probe volume and of second measurement data obtained in a second optical coherence measurement in a second probe volume, wherein between the first optical coherence measurement and the second optical coherence measurement, an ambient pressure to which the sample is exposed is changed by the ambient pressure modulator.

11. The apparatus according to claim 10, wherein the measurement cavity has a proximal opening circumferentially delimited by a sealing member for sealing a closed volume jointly defined by the measurement cavity and a peripheral region adjacent the sample, by forming a circumferential sealing connection between the sealing member and the peripheral region.

12. The apparatus according to claim 10 or 11, wherein the ambient pressure modulator has a precision of 0.5 mbar or less in adjusting the ambient pressure, in particular of 0.1 mbar or less, more particularly wherein the ambient pressure modulator has an accuracy of 0.5 mbar or less, in particular of 0.1 mbar or less in adjusting the ambient pressure.

13. The apparatus according to one of claims 10 to 12, comprising a sample-to-beam aligner for effecting that the first probe volume and the second probe volume are strongly overlapping, e.g., via one or more of
- tracking a motion of the sample and carrying out the second optical coherence measurement at a time when a position of the sample deviates from the position of the sample during the first optical coherence measurement by less than a threshold position difference;
- tracking a motion of the sample and steering a sample beam used in the second optical coherence measurement to interact with the sample in a location coinciding, within a threshold location difference, with s location in which the sample beam did interact with the sample during the first optical coherence measurement;
- influencing a motion of the sample to make the sample return towards, in particular into the position it had during the first optical coherence measurement.

14. The apparatus according to one of claims 10 to 13, comprising a control unit configured to control the optical coherence measurement device and the ambient pressure modulator in such a way that
(A) the first optical coherence measurement is carried out by the optical coherence measurement device, while the sample is exposed to an ambient pressure amounting to a pressure p1, in particular wherein the ambient pressure is adjusted to amount to the pressure p1 by the ambient pressure modulator; and subsequently
(B) an ambient pressure to which the sample is exposed is changed by the ambient pressure modulator from the pressure p1 to a pressure p2 which is different from the pressure p1; and subsequently
(C) the second optical coherence measurement is carried out by the optical coherence measurement device;
in particular wherein the control unit is furthermore configured to control the evaluation unit in such a way that subsequently,
(D) an evaluation in dependence of both, the first and the second measurement data, is carried out by the evaluation unit.
